(19) Europäisches Patentamt
European Patent Office
Office européen des brevets

(11) Numéro de publication: **0 099 297**
**B1**

(12) # FASCICULE DE BREVET EUROPEEN

(45) Date de publication du fascicule du brevet: **06.04.88**

(51) Int. Cl.⁴: **C 07 D 501/36,** A 61 K 31/545 // C07D277/20

(21) Numéro de dépôt: **83401413.6**

(22) Date de dépôt: **08.07.83**

(54) **Nouveaux dérivés des céphalosporines, leur procédé de préparation et médicaments antibiotiques contenant lesdits dérivés.**

(30) Priorité: **13.07.82 FR 8212317**

(43) Date de publication de la demande: **25.01.84 Bulletin 84/04**

(45) Mention de la délivrance du brevet: **06.04.88 Bulletin 88/14**

(84) Etats contractants désignés: **AT BE CH DE FR GB IT LI LU NL SE**

(56) Documents cités:
**FR-A-2 387 234**
**FR-A-2 442 240**
**FR-M- 2 030**

**Le dossier contient des informations techniques présentées postérieurement au dépôt de la demande et ne figurant pas dans le présent fascicule.**

(73) Titulaire: **SANOFI, société anonyme**
**40, Avenue George V**
**F-75008 Paris (FR)**

(72) Inventeur: **Labeeuw, Bernard**
**22 rue Paul Eluard**
**F-34100 Montpellier (FR)**
Inventeur: **Salhi, Ali**
**639 rue de Valène**
**F-34980 St-Gely-du-Fesc (FR)**

(74) Mandataire: **Gillard, Marie-Louise et al**
**Cabinet Beau de Loménie 55, Rue d'Amsterdam**
**F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 099 297 B1

**Description**

La présente invention concerne des dérivés de la famille des céphalosporines, leur procédé de préparation et leur application thérapeutique.

Les composés selon l'invention répondent à la formule:

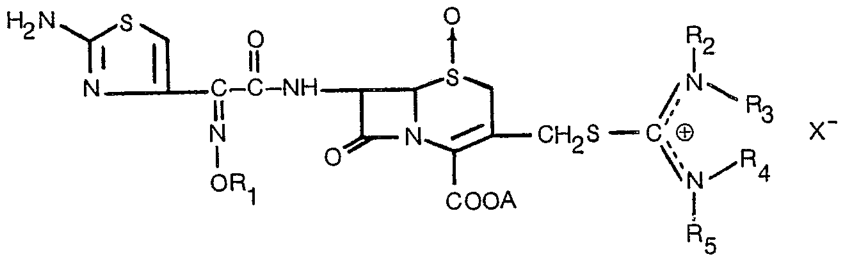

(I)

dans laquele: — le groupe

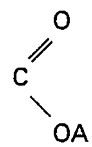

en position 4 est un radical acide ou un sel alcalin ou alcalino-terreux ou un sel d'amine, par exemple la triéthylamine ou les éthanolamines ou encore un radical ester facilement hydrolysable ou métabiliquement labile et pharmaceutiquement acceptable;

— $R_1$ représente un groupe 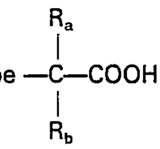

dans lequel $R_A$ et $R_B$ désignent chacun indépendamment l'hydrogéne, ou un group alkyle inférieur, de préférence un groupe méthyle,

ou $R_A$ et $R_B$ pris ensemble avec l'atome de carbone auquel ils sont liés, forment un groupe cyclobutyle,

—$R_2$ et $R_3$ désignent chacun indépendamment l'hydrogéne, ou un group alkyle inférieur ou un groupe alcényle en $C_4$. Par alkyle inférieur, on entend un alkyle comprenant de 1 à 4 atomes de carbone.

—$R_4$ et $R_6$ désignent chacun indépendamment l'hydrogéne, un groupe alkyle en $C_1$—$C_4$, un groupe alcényle en $C_4$ ou l'un d'entre eux est l'hydrogène et l'autre est un groupe propèn-2-yl ou encore.

$R_4$ et $R_5$ pris ensemble avec l'atome d'azote auquel ils sont liés forment un noyau cyclique à 5 ou 6 chaînons pouvant comporter un second atome d'azote, en particulier les cycles suivants:

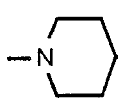 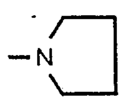 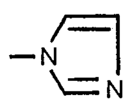 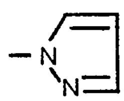

— $X^-$ désigne l'anion d'un acide ou (I) est sous forme de betaïne

La double liaison de la fonction isothiouronium peut être délocalisée comme cela est représenté sur la formule du composé (1).

Par suite de la présence dans la formule d'un groupement oxime, les composés (1) existent sous deux formes isomères syn et anti. Les isomères syn dont l'activité thérapeutique est supérieure sont les composés préférés.

Il est entendu que les composés (I) indiqués ci-dessus peuvent exister:

— soit sous la forme indiquée dans la formule (I)

— soit sous la forme tautomère (I'):

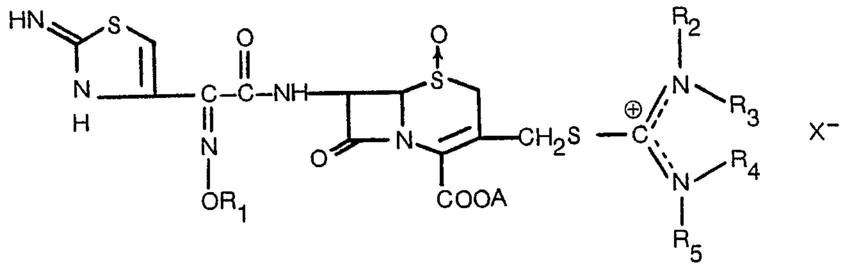

(I′)

dans laquelle A, $R_1$, $R_2$, $R_3$, $R_4$, $R_5$ on les significations indiquées précédemment.

L'invention concerne également un procédé de préparation des composés de formule (I).

Ce procédé consiste à acyler tout d'abord l'amino-7 bromo-méthyl-3 céphème-3 carboxylate de tertiobutyle-4 S-oxyde (II) par l'acide (III) selon la schéma réactionnel suivant dans lequel $R'_1$ représente l'ester t-butylique correspondant à $R_1$.

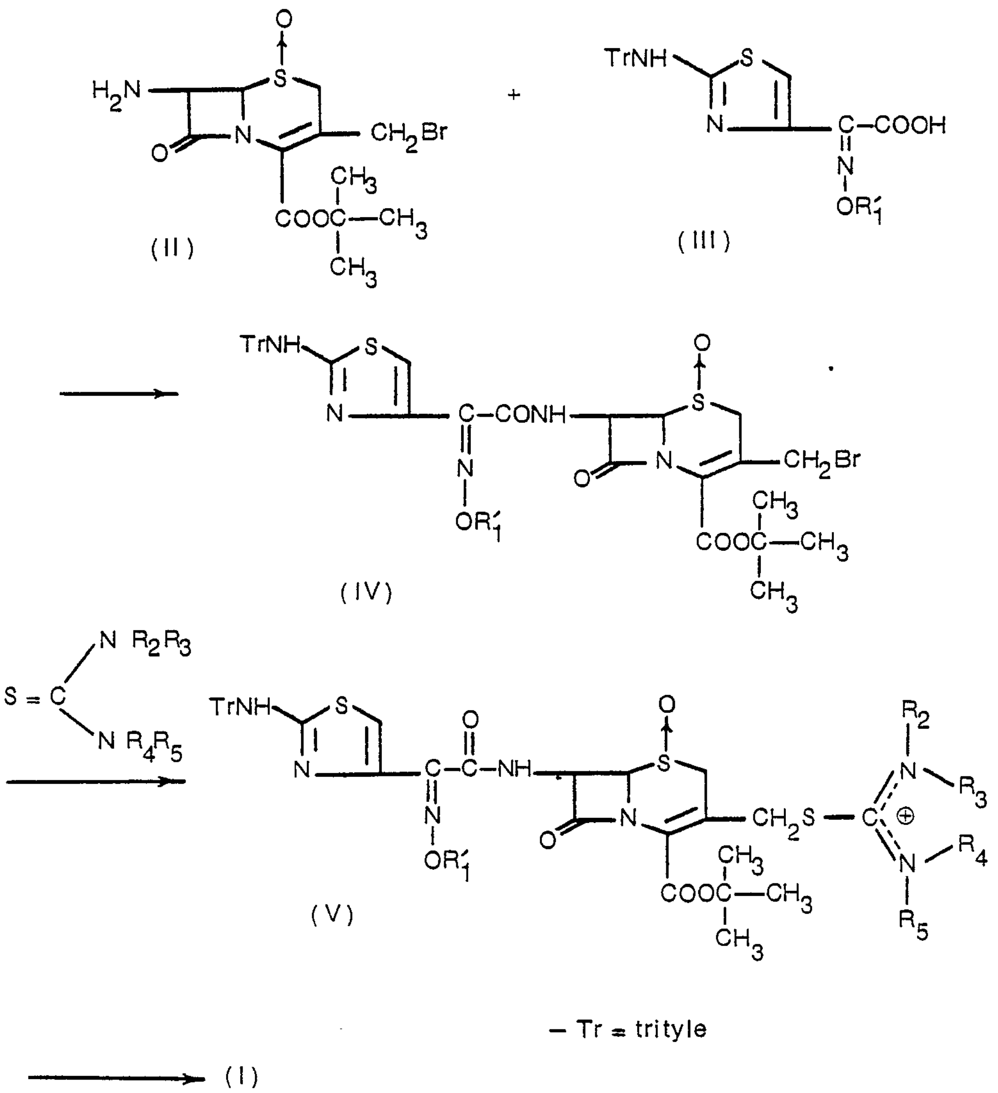

(II) (III) (IV) (V)

— Tr = trityle

(I)

Avant d'effectuer la réaction d'acylation, il est souhaitable de substituer le groupe amino de l'acide par un groupe protecteur facile à éliminer ultérieurement. On peut utiliser les groupes habituellement utilisés en synthèse organique pour la protection des groupes aminés et, en particulier, le groupe trityle.

De même, lorsque le substituant $R_1$ de l'acide (III) comporte un groupe carboxylique, il est nécessaire de transformer celui-ci en ester. On choisit de préférence un ester suffisamment labile pour pouvoir régénérer la fonction acide en fin de réaction. On utilise le plus souvent l'ester tertiobutylique.

Pour effectuer la réaction d'acylation, il est nécessaire de procéder à l'activation du groupe carboxyle du composé (III), de préférence par transformation en anhydride à l'aide d'un carbodimide, en général le dicyclohexylcarbodiimide.

La réaction d'activation est effectuée au sein d'un solvant organique convenable tel que le tétrahydrofuranne à une température comprise entre 0 et 50°C et, le préférence, à température ambiante. La réaction d'activation est éventuellement facilitée par addition d'un dérivé hydroxylé tel que l'hydroxy-1 benzotriazole.

La solution du réactif d'acylation ainsi obtenue, débarrassée par filtration de la dicyclohexylurée formée, est ajoutée à une solution du composé (II) dans un solvant tel que le diméthylformamide. L'addition des deux réactifs peut aussi s'effectuer dans l'ordre inverse.

L'acylation peut également s'effectuer par le chlorure d'acide de (III):

(II) +

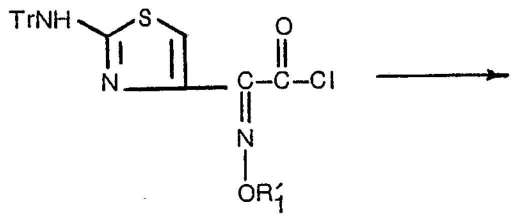

(IV)

On ajoute au composé (IV) ainsi obtenu la thiourée

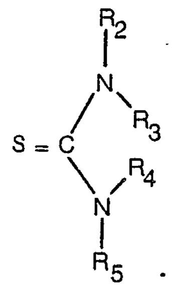

où $R_2$, $R_3$, $R_4$, $R_5$ on les valeurs désignées précédemment.

On opère dans un solvant convenable tel que le diméthylformamide ou le N,N-diméthylacétamide en présence d'une base comme la triéthylamine.

Enfin, pour aboutir au composé (I), le groupe protecteur sur l'amine et le ou les groupes esters tertiobutyliques sont éliminés par un procédé connu, en particulier par hydrolyse en milieu acide en utilisant un acide organique tel que l'acide formique ou l'acide trifluoroacétique ou un mélange acide chlorhydrique — acide acétique.

En ce qui concerne les matières premières de la réaction, les composés (II) et le composé (III) ainsi que ses dérivés dans lesquels le groupe aminé est bloqué par un groupe protecteur sont connus.

Préparation des thiourées:

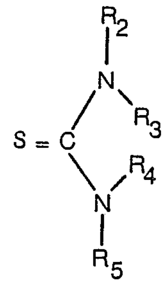

Les thiourées dans lesquelles $R_2$, $R_3$, $R_4$, $R_5$ représentent des groupes alkyle ou alcenyle sont préparées selon les méthodes décrites dans: E. H. RODD, Chemistry of Aliphatic compounds, vol. IB. Elsevier, 1952, 924—929.

Les thiourées dans lesquelles $R_4$ et $R_5$ pris ensemble avec l'atome d'azote auquel ils sont liés forment un noyau cyclique à 5 ou 6 chaînons sont préparées à partir du chlorure de diméthylthiocarbamoyle ou d'isothiocyanate de méthyle sur lequel on fait réagir l'hétérocycle azoté selon le schéma réactionnel ci-dessous:

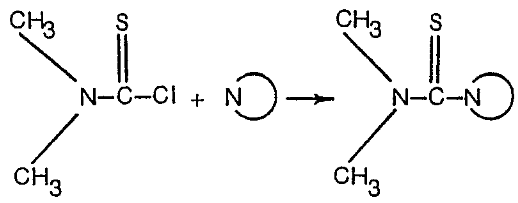

où

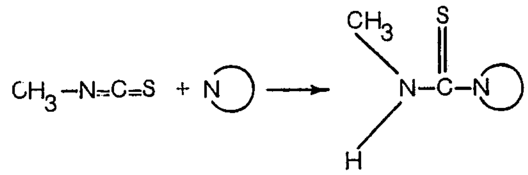

Les composés (I) de l'invention dans lesquels A est autre que H s'obtiennent à partir des composés (I) dans lesquels A est H par des réactions connues en elles-mêmes.

Ainsi, les sels minéraux sont obtenus par action sur les composés (I) dans lesquels A est H d'une base minérale telle que la soude ou la potasse ou le bicarbonate de sodium en quantité équimoléculaire; la réaction de salification est réalisée dans un solvant tel que l'eau ou l'éthanol et le sel obtenu est isolé par évaporation de la solution.

Les sels de bases organiques sont obtenus par action, sur solution de l'acide (I, A = H) dans un solvant ou un mélange de solvants convenables, d'une quantité équimoleculaire de la base organique. Les sel est isolé par précipitation avec l'éther.

Les esters sont obtenus par les procédés connus d'estérification; par exemple, on utilisera avantageusement l'action d'un dérivé halogèné sur un sel tel que le sel de sodium d'acide; on réalisera de préférence la réaction dans un solvant capable de dissoudre le dérivé acide de départ, par exemple dans le diméthylformamide.

Les isomères de forme syn et anti s'obtiennent par un choix convenable des réactifs ou des conditions expérimentales.

Les exemples suivants permettent de mieux comprendre la portée de l'invention.

Ainsi qu'il est habituel dans cette famille de composés, les produits suivant l'invention ne présentent pas de point de fusion net, mais seulement des points de décomposition ne permettant pas de les caractériser.

Les produits seront donc caractérisés par leur spectre de résonance magnétique nucléaire enregistré à 60 MHz ou à 250 MHz, l'étalon interne étant l'hexaméthyldisiloxanne.

Les spectres sont enregistrés dans le diméthylsulfoxyde deutérié.

Les abréviations suivantes seront utilisées:

— S: singulet
— D: doublet
— D de D: doublet de doublet
— Se.: singulet élargi
— M: multiplet
— Q: quadruplet
— AB: système AB
— J: représente la constante de couplage.

De plus, les microanalyses élémentaires ont été effectuées dans chaque cas et sont en accord avec les formules indiquées.

Exemple 1

Trifluoracétate de l'acide [(amino-2 thiazolyl-4)-2 (carboxy-2 propyl-2 oxyimino)-2 acétamido]-7 (N,N,N',N'-tétraméthyl uronium thiométhyl)-3 céphème-3 carboxylique-4 S-oxyde-1, isomére syn.

(I) $R_1 = -C(CH_3)_2CO_2H$; $R_2 = R_3 = R_4 = R_5 = CH_3$ $X = CF_3CO_2-$

CM 41 089.

a) — [(tritylamino-2 thiazolyl-4)-2 (t. butoxycarbonyl-2 propyl-2 oxyimino)-2 acétamido]-7 brométhyl-3 céphème-3 carboxylate de t-butyle-4 S-oxyde-1, isomère syn

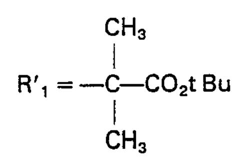

(IV)

A 830 mg de chlorhydrate d'amino-7 bromométhyl-3 céphème-3 carboxylate-4 de t-butyle S-oxyde-1 dissous dans 15 ml de chlorure de méthylène, on ajoute 209 mg de l'acide tritylamino-2 thiazolyl-4)-2 (terbutoxy carbonyl-2 propyl-2 oxyimino)-2 acétique, 422 mg de dicyclohexylcarbodiimide et 10 mg d'hydroxy-1 benzotriazole.

Après 4 heures d'agitation à température ambiante, on filtre la dicyclohexylurée, on évapore le chlorure de méthylène sous vide, dissout le résidu dans l'éther, lave avec une solution d'acide chlorhydrique normale puis à l'eau, puis avec une solution saturée de carbonate acide de sodium puis à l'eau. On sèche la phase éthérée sur sulfate de magnésium puis concentre sous vide. On chromatographie sur 80 g de gel de silice et élue avec le mélange hexane-acétate d'éthyle à 60/40 (vol/vol). L'éluat est

évaporé pour obtenir 650 mg de a).

Autre préparation de a)

On prépare une suspension de 20 g d'acide (tritylamino-2 thiazolyl-4)-2 (t-butoxycarbonyl-2 propyl-2 oxyimino)-2 acétique dans 100 ml de chlorure de méthylène refroidi à 0°—2°C. On ajoute lentement 7,3 g de $PCl_5$ et on agite pendant 30 minutes à cette température. On verse 1 litre d'hexane pour précipiter puis on filtre et sèche sous vide pour obtenir 21,2 g de chlorure d'acide (tritylamino-2 thiazolyl-4)-2 (t-butoxycarbonyl-2 propyl-2 oxyimino)-2 acétique. Point de fusion: 135°C.

Spectre de RMN dans le deutérochloroforme

15H à 7,40 ppm (H du trityle, S) — 1H à 6,42 ppm (H du thiazole, S) — 6H à 1,67 ppm ($C(CH_3)_2$, S) — 9H à 1,45 ppm ($C(CH_3)_3$, S).

A une suspension de 1,5 g de chlorhydrate de l'amino-7 bromométhyl-3 céphème-3 carboxylate de t-butyle-4 S-oxyde dans 30 ml de chlorure de méthylène anhydre, on ajoute à 5°C 2,2 g de chlorure d'acide précédemment préparé et 1 ml de N,N-diméthylaniline. On laisse revenir à température ambiante; après 2 h 30 d'agitation à l'ambiance, on verse sur 100 ml d'éther isopropylique pour précipiter, filtre, lave à l'éther isopropylique puis à l'hexane et sèche sous vide pour obtenir 3 g de a).

b) — Bromure de [(tritylamino-2 thiazolyl-4)-2 (t-butoxy carbonyl-2 propyl-2 oxyimino)-2 acétamido]-7, N,N,N',N'-tétraméthyl uronium thiométhyl-3 céphème-3 carboxylate de t-butyle-4 S-oxyde-1, isomère syn.

On abandonne 2 jours à 5°C une solution de 1 g de a) et 0,21 g de N,N'-tétraméthyl thiourée dans 5 ml de N,N-diméthylacétamide. La solution obtenue est versée goutte à goutte sur 100 ml d'éther isopropylique en agitant.

Le solide obtenu est redissous dans 5 ml de chlorure de méthylène puis chromatographié sur 25 g de gel de silice, l'éluant est un mélange de chlorure de méthylène-méthanol 90/10 (vol/vol).

On obtient 0,7 g du produit b)

c) — CM 41 089.

On abandonne à température ambiante pendant 45 minutes, une solution de 0,62 g de b) dans 4 ml d'acide trifluoracétique. On concentre sous vide puis on précipite par addition d'éther, on filtre puis on lave à l'éther et sèche sur l'anhydride phosphorique pour obtenir 0,48 g de CM 41 089.

Spectre de RMN.

1H à 8,5 ppm (CONH, D, J=9Hz) — 1H à 6,85 ppm (H du thiazole, S) — 1H à 5,95 ppm ($H_7$, D de D, J=9Hz, J=4Hz) — 1H à 5,02 ppm ($H_6$, D, J=4Hz) — 1H à 4, 10 ppm ($CH_2S$, AB, $J_{AB}$=13Hz) — 1H à 3,90 ppm ($CH_2S$, AB, $J_{AB}$=13Hz) — 1H à 3,80 ppm ($CH_2SO$, AB, $J_{AB}$=17Hz) — 1H à 3,70 ppm ($CH_2SO$, AB, $J_{AB}$=17Hz) — 12H à 3,20 ppm ($2(CH_3)_2N$, S) — 6H à 1,45 ppm ($(CH_3)_2C$, S).

## Example 2

Trifluoracétate de l'acide [(amino-2 thiazolyl-4)-2 carboxy-2 éthyl-2 oxyimino)-2 acétamido]-7 (N,N,N',N'-tétraméthyluronium thiométhyl)-3 céphème-3 carboxylique-4 S-oxyde-1-isomère syn

SR 41 361 A

Ce produit est préparé de la même manière et dans les mêmes conditions opératoires que le CM 41 089, à partir de l'acide (tritylamino-2 thiazolyl-4)-2 (t-butoxycarbonyl-1 éthyl-1 oxyimino)-2 acétique.

Le SR 41 361 A, mélange de 2 diastéréoisomères, est identifié par son spectre.

Spectre de RMN

1H à 8,80 ppm (CONH, 2D, J=9Hz) — 2H à 7,40 ppm ($NH_2$ du thiazole, Se.) — 1H à 6,80 ppm (H du thiazole, 2 S) — 1H à 5,96 ppm ($H_7$, M) — 1H à 5,00 ppm ($H_6$, M) — 1H à 4,20 ppm

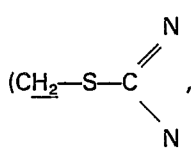

D, J=13Hz) — 1H à 4,55 ppm ($CHCH_3$, M) — 1H à 3,85 ppm

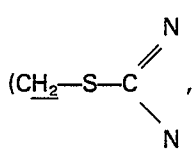

D, J = 13 Hz) — 2H à 3,75 ppm ($CH_2$—SO, M) — 12H à 3,15 ppm $(CH_3)_2N$, Se.) — 3H à 1,40 ppm ($CH_3$—CH, D, J=7Hz).

Exemple 3

Trifluoracétate de l'acide [(amino-2 thiazolyl-4)-2 carboxy-2 propyl-2 oxyimino)-2 acétamido]-7 (N-méthyl, N',N'-pentaméthylène uronium thiométhyl)-3 céphème-3 carboxylique-4 S-oxyde-1, isomère syn

SR 41 381 A

Ce produit est obtenu selon la méthode décrite précédement, à partir du composé a) de l'exemple 1 et de N-méthyl, N',N'-pentaméthylène thiourée préparèe comme suit:

A 2,7 ml de pipéridine refroidie à −30°C, on ajoute lentement 2 g d'isothiocyanate de méthyle, on dilue avec 10 ml de chlorure de méthylène, on agite 1 heure puis on évapore à sec sous vide. Le résidu est trituré avec de l'éther puis filtré. Après séchage, on obtient 4 g de N-méthyl, N',N'-pentaméthylène thiourée. Point de fusion 130°C.

Le SR 41 381 A est identifié par son spectre de RMN. 1H à 9,50 ppm (N<u>H</u> $CH_3$, Se.) — 1H à 8,47 ppm (CON<u>H</u>, D, J=9Hz) 2H à 7,50 ppm ($NH_2$, Se.) — 1H à 6,90 ppm (<u>H</u> du thiazole, S) — 1H à 5,96 ppm ($H_7$, D de D, $J_1$=9Hz, $J_2$=4Hz) — 1H à 5,0 ppm ($H_6$, D, J=4Hz) — 2H à 4,0 ppm

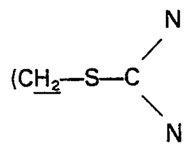 ,

AB, $J_{AB}$=13Hz) — 2H à 3,80 ppm ($CH_2$—SO, AB, $J_{AB}$=17Hz) — 4H à 3,60 ppm

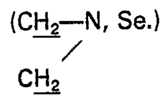

3H à 3,0 ppm ($C\underline{H_3}N$, S) 6H à 1,55 ppm ($—C\underline{H_2}—C\underline{H_2}—CH_2$, Se.) — 6H à 1,45 ppm ($(C\underline{H_3})_2—C$, S).

Exemples 4 à 13

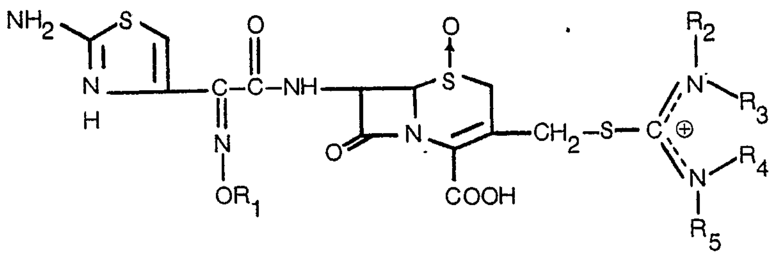 $CF_3COO^-$

(I) Isomère syn

Les significations de $R_1$, $R_2$, $R_3$ et N sont données dans le tableau I

TABLEAU I

| N° de Code | $R_1$ | $R_2$ | $R_3$ | 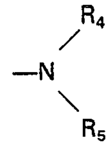 |
|---|---|---|---|---|
| SR 41 362 A | 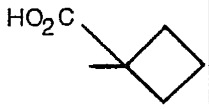 | $-CH_3$ | $-CH_3$ | 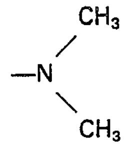 |
| SR 41 363 A | 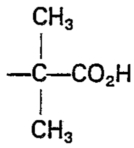 | H | H | $-NH_2$ |
| SR 41 380 A | 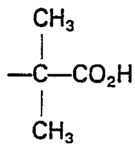 | $-CH_3$ | $-CH_3$ | 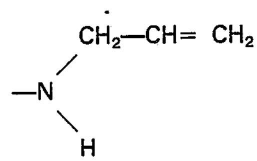 |
| SR 41 382 A | 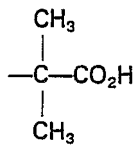 | $CH_3$ | $CH_3$ | 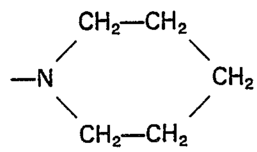 |
| SR 41 383 A | 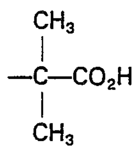 | —H | $-CH_3$ | 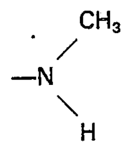 |
| SR 41 384 A | 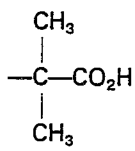 | $-CH_3$ | $-CH_3$ | 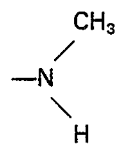 |
| SR 41 385 A | 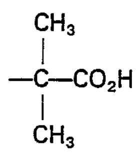 | $C_2H_5$ | $C_2H_5$ | 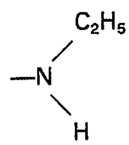 |
| SR 41 605 A | 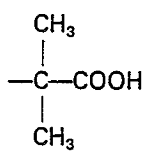 | $-CH_3$ | $-CH_3$ | $-NH-C_2H_5$ |
| SR 41 609 A | 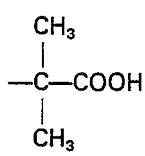 | H | $-CH_3$ | 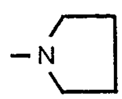 |
| SR 41 912 A | 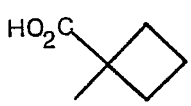 | $-CH_3$ | $-CH_3$ | $-NH-CH_3$ |

Spectres de RMN des exemples 4 à 13

| N° de Code | Spectre de RMN |
|---|---|
| SR 41 362 A | 1H à 8,80 ppm ($CON\underline{H}$, D, J: 9Hz) —<br>2H à 7,50 ppm ($N\underline{H_2}$ du thiazole, Se.) —<br>1H à 6,80 ppm (H thiazole, S)<br>1H à 5,96 ppm ($H_7$, D de D, $J_1$=9Hz, $J_2$=4Hz) —<br>1H à 5,0 ppm ($H_6$, D, J=4Hz) —<br>1H à 4,10 ppm ($C\underline{H_2}$—S—C 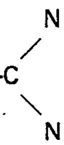 , D, J=13Hz) —<br>1H à 3,90 ppm ($C\underline{H_2}$—S—C 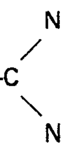 , D, J=13Hz) —<br>2H à 3,80 ppm ($C\underline{H_2}SO$, AB, $J_{AB}$=17Hz) —<br>12H à 3,10 ppm (—C—($N(C\underline{H_3})_2)_2$, Se.) —<br>4H à 2,40 ppm ( 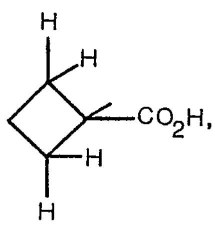 M en α du cyclobutyle) —<br>2H à 1,85 ppm ( 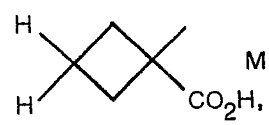 M en β du cyclobutyle) |
| SR 41 363 A | 4H à 9,40 ppm (C 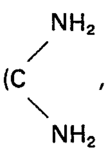 , Se.)<br>1H à 8,40 ppm ($CON\underline{H}$, D, J=9Hz) —<br>2H à 7,40 ppm ($NH_2$ du thiazole, Se.) —<br>1H à 6,90 ppm (H du thiazole, S) —<br>1H à 6,0 ppm ($H_7$, D de D, $J_1$=9Hz, $J_2$=4Hz) —<br>1H à 5,0 ppm ($H_6$, D, J=4Hz) —<br>2H à 4,10 ppm ($C\underline{H_2}$—S—C 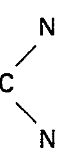 , AB, $J_{AB}$=13Hz) |

| | |
|---|---|
| SR 41 363 A | 2H à 3,80 ppm ($C\underline{H_2}$—SO, — AB, $J_{AB}$—17Hz) —<br>6H à 1,45 ppm ($C(C\underline{H_3})_2$, S) |
| SR 41 380A | 1H à 9,50 ppm ($N\underline{H}$, Se.) —<br>1H à 8,50 ppm ($NON\underline{H}$, D, J=9Hz) —<br>2H à 7,50 ppm ($NH_2$ du thiazole, Se.) —<br>1H à 6,90 ppm (H du thiazole, S) —<br>1H à 5,96 ppm ($H_7$, D de D, $J_1$=9Hz, $J_2$=4Hz) —<br>1H à 5,80 ppm (—$CH_2$—$C\underline{H}$=$CH_2$, M) —<br>2H à 5,10 ppm (—$CH_2$—CH=$C\underline{H_2}$, M) —<br>1H à 5 ppm ($H_6$, D, J=4Hz) —<br>3H à 4,10 ppm ($C\underline{H_2}$—S et $CH_2$—N, M) —<br>1H à 3,95 ppm ($C\underline{H_2}$—S, D, J=13Hz) —<br>2H à 3,90 ppm ($C\underline{H_2}$—SO, AB, J=17Hz) —<br>6H à 3,20 ppm ($(CH_3)_2N$, S) —<br>6H à 1,45 ppm ($(C\underline{H_3})_2C$—$CO_2H$, S) |
| SR 41 382 A | 1H à 8,47 ppm ($N\underline{H}CO$, D, J=9Hz) —<br>2H à 7,40 ppm ($N\underline{H_2}$ du thiazole, Se.) —<br>1H à 6,90 ppm (H du thiazole, S) —<br>1H à 5,95 ppm ($H_7$, D de D, $J_1$=9Hz, $J_2$=4Hz)<br>1H à 5,6 ppm ($H_6$, D, J=4Hz)—<br>N<br>/<br>2H à 4,0 ppm ($C\underline{H_2}$—S—C , AB, $J_{AB}$=13Hz) —<br>\\<br>N<br>2H à 3,80 ppm ($C\underline{H_2}SO$, AB, $J_{AB}$=17Hz) —<br>4H à 3,55 ppm ($C\underline{H_2}N$, Se.) —<br>6H à 3,10 ppm ($(C\underline{H_3})_2N$, S) —<br>6H à 1,55 ppm ($CH_2$—$CH_2CH_2$, SE.) —<br>6H à 1,45 ppm ($C(C\underline{H_3})_2$, S) |
| SR 41 383 A | 1H à 9,50 ppm ($N\underline{H}$ $CH_3$, Se.) —<br>1H à 9,40 ppm ($N\underline{H}$ $CH_3$ Se.) —<br>1H à 8,47 ppm ($CON\underline{H}$, D, J=9Hz) —<br>2H à 7,40 ppm ($N\underline{H_2}$ du thiazole Se.) —<br>1H à 6,90 ppm (H du thiazole, S) —<br>1H à 5,95 ppm ($H_7$, D de D, $J_1$=4Hz, $J_2$=9Hz) — |

| | |
|---|---|
| R 41 383 A | 1H à 5,0 ppm ($H_6$, D, J=4Hz) —<br>2H à 4,20 ppm ($C\underline{H}_2$—S—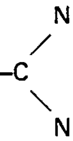 , AB, $J_{AB}$=13Hz —<br>2H à 3,80 ppm ($C\underline{H}_2SO$, AB, $J_{AB}$=17Hz) —<br>3H à 2,95 ppm ($CH_3N$, S) —<br>3H à 2,80 ppm ($CH_3N$, S) —<br>6H à 1,45 ppm ($(CH_3)_2C$, S) |
| SR 41 384 A | 1H à 9,40 ppm (N$\underline{H}$ Se) —<br>1H à 8,50 ppm (CON$\underline{H}$, D, J=9Hz) —<br>2H à 7,40 ppm ($N\underline{H}_2$ du thiazole, Se.) —<br>1H à 6,90 ppm (H du thiazole, S) —<br>1H à 5,96 ppm ($H_7$, D de D, $J_1$=9Hz, $J_2$=4Hz) —<br>1H à 5,00 ppm ($H_6$, D, J=4Hz) —<br>2H à 4,00 ppm ($C\underline{H}_2$—S—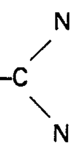 , AB, $J_{AB}$=13Hz) —<br>2H à 3,80 ppm ($C\underline{H}_2$—SO—, AB, $J_{AB}$=17Hz) —<br>6H à 3,25 ppm ($(C\underline{H}_3)_2N$, Se.) —<br>3H à 3,05 ppm ($CH_3NH$, Se.) —<br>6H à 1,45 ppm ($(CH_3)_2C$, S). |
| SR 41 385 A | 1H à 9,20 ppm (N$\underline{H}$ $C_2H_5$, Se.) —<br>1H à 8,50 ppm (CON$\underline{H}$, D, J=9Hz) —<br>2H à 7,50 ppm ($N\underline{H}_2$ du thiazole, Se.) —<br>1H à 6,80 ppm ($\underline{H}$ du thiazole, S) —<br>1H à 5,96 ppm ($H_7$, D de D, $J_1$=9Hz, $J_2$=4Hz) —<br>1H à 5,00 ppm ($H_6$, D, J=4Hz) —<br>2H à 4,10 ppm ($C\underline{H}_2$—S—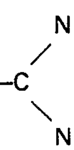 , AB, $J_{AB}$=13Hz) —<br>2H à 3,8 ppm ($C\underline{H}_2$—SO, AB, $J_{AB}$=17Hz)<br>6H à 3,50 ppm ($C\underline{H}_2N$, M) —<br>6H à 1,45 ppm ($(C\underline{H}_3)_2C$, S)<br>9H à 1,20 ppm ($C\underline{H}_3CH_2N$, M) |

| | |
|---|---|
| SR 41 605 A | 1H à 9,25 ppm ($=\overset{\oplus}{N}H$—, Se) — |
| | 1H à 8,50 ppm (—N<u>H</u>—CO, D, J=9Hz) —<br>1H à 6,80 ppm (H thiazole, S) —<br>1H à 5,95 ppm ($H_7$, D de D, $J_1$ = 9Hz, $J_2$ = 4Hz)<br>1H à 5,0 ppm ($H_6$, D, J=4Hz) —<br>1H à 4,10 ppm (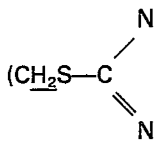, D, J=13 Hz —<br>1H à 3,95 ppm (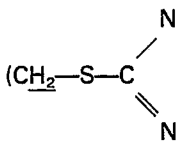, D, J=13Hz) —<br>1H à 3,90 ppm (— 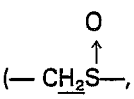, D, J=17Hz) —<br>1H à 3,75 ppm (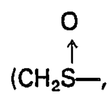, D, J = 17 Hz) —<br>2H à 3,50 ppm ($CH_3$—C<u>$H_2$</u>—N, M) —<br>6H à 3,20 ppm (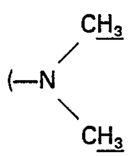, S.e.) —<br>6H à 1,45 ppm (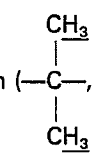, S) —<br>3H à 1,12 ppm (—N—$CH_2$—C<u>$H_3$</u>, T, J=7Hz). |
| SR 41 609 A | 1H à 9,10 ppm (N<u>H</u>—$CH_3$, S.e.) —<br>1H à 8,45 ppm (N<u>H</u>—CO, D, J=9Hz) —<br>1H à 6,75 ppm (H thiazole, S) —<br>1H à 6,0 ppm ($H_7$, D de D, $J_1$=9Hz, $J_2$=4Hz) —<br>1H à 5,0 ppm ($H_6$, D, J=4Hz) —<br>1H à 4,15 ppm (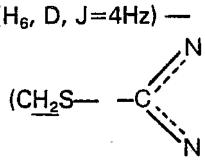, D, J=13Hz) —<br>2H à 3,95 ppm (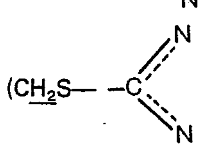, et 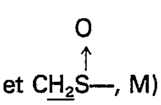, M) — |

| | |
|---|---|
| SR 41 609 A | O<br>↑<br>1H à 3,80 ppm ($C\underline{H_2}S$, D, J=17Hz)<br>4H à 3,40 ppm ( 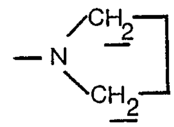 , M) —<br>3H à 3,05 ppm (NH—$C\underline{H_3}$, S.e.) —<br>4H à 1,90 ppm ( 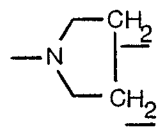 , M) —<br>6H à 1,45 ppm ( 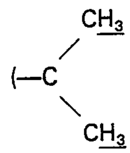 , S.e.). |
| SR 91 912 A | 1H à 6,90 ppm (H thiazole, S) —<br>1H à 5,95 ppm ($H_7$, M) —<br>1H à 5,0 ppm ($H_6$, M) —<br>6H à 3,15 ppm ( 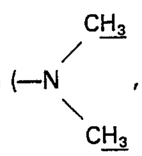 , S.e.) —<br>3H à 3,05 ppm (NH—$C\underline{H_3}$, D, J=7Hz) —<br>4H à 2,40 ppm ( 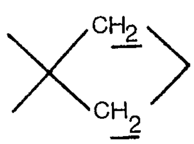 , M) —<br>2H à 1,90 ppm ( 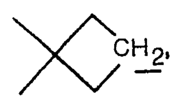 , M). |

Les produits de l'invention peuvent donc être utilisés comme antibiotiques en médicine humaine ou vétérinaire. Ils peuvent être utilisés dans toutes les infections bactériennes à germes sensibles.

Les produits de l'invention ont été étudiés en ce qui concerne leurs propriétés pharmacologiques et, plus spécialement, l'action bactériostatique.

L'action bactériostatique in vitro a été déterminée en milieu solide par la méthode des dilutions.

Les résultats exprimés en concentrations minimales inhibitrices (CMI — µg/ml) concernent les résultats obtenus sur différentes souches.

Ces résultats sont rassemblés dans le tableau II.

TABLEAU II

| Souches / Produits | Escherichia Coli R 69/3 Tem | Proteus 1510 | Klebsiella RO 30 | Enterobacter P 99 |
|---|---|---|---|---|
| CM 41 089 | 0,25 | ≤0,125 | 0,5 | 1 |
| SR 41 361 A | 0,5 | 0,5 | 0,5 | 1 |
| SR 41 362 A | 0,25 | ≤0,125 | 0,5 | 2 |
| SR 41 363 A | 0,5 | 4 | 2 | 16 |
| SR 41 380 A | 0,25 | ≤0,125 | 0,5 | 1 |
| SR 41 381 A | ≤0,125 | ≤0,125 | 0,5 | 1 |
| SR 41 382 A | 0,25 | ≤0,125 | 0,5 | 1 |
| SR 41 383 A | 0,5 | 1 | 2 | 8 |
| SR 41 384 A | 0,25 | ≤0,125 | 0,5 | 1 |
| SR 41 385 A | ≤0,125 | ≤0,125 | 0,5 | 2 |
| SR 41 605 A | 0,25 | ≤0,125 | 0,5 | 0,5 |
| SR 41 609 A | ≤0,125 | ≤0,125 | 0,5 | 0,5 |
| SR 41 912 A | ≤0,125 | ≤0,125 | 0,5 | 2 |

Les essais effectués sur les animaux n'ont mis en évidence aucune toxicité des produits selon l'invention.

Les compositions pharmaceutiques sont réalisées à partir des composés (I) sous leur forme acide ou, lorsque leur solubilité est insuffisante, sous forme d'un sel.

Les compositions pharmaceutiques peuvent être solides ou liquides et se présenter, par exemple, sous forme de comprimés, gélules, granulés, pommades, crèmes, gels ou préparations injectables.

La posologie peut varier dans de larges proportions, en particulier suivant le type et la gravité de l'infection à traiter et suivant le mode d'administration. Le plus souvent, chez l'adulte par voie injectable, elle est comprise entre 0,250 g et 4 g par jour.

A titre d'exemple de composition pharmaceutique, on peut préparer des ampoules contenant:

| | |
|---|---|
| CM 41 089 | 1 g |
| L-Lysine | 0,212 g |
| Eau pour préparation injectable | 4 ml |

**Revendications pour les Etats contractants: BE CH DE FR GB IT LI LU NL SE**

1. Dérivés des céphalosporines de formule

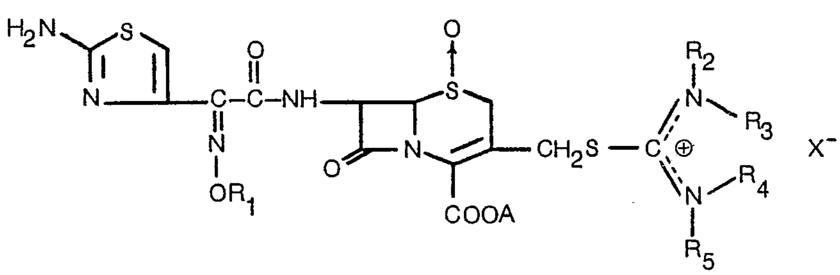

(I)

sous forme d'isomère syn, d'isomère anti ou d'un mélange de ces isomères, dans laquelle le groupe acide-COOA est sous forme acide ou sous forme de sel ou sous forme d'ester facilement hydrolysable,

— $R_1$ représente un groupe 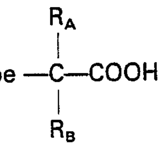

dans lequel $R_A$ et $R_B$ désignent chacun indépendamment l'hydrogène ou un groupe alkyle en $C_1$—$C_4$, de préférence un groupe méthyle, ou $R_A$ et $R_B$ pris ensemble avec l'atome de carbone, auquel ils sont liés, forment un groupe cyclobutyle;

— $R_2$ et $R_3$ désignent chacun indépendamment l'hydrogène un groupe alkyle en $C_1$—$C_4$ ou un groupe alcényle en $C_4$;

— $R_4$ et $R_5$ désignent chacun indépendamment l'hydrogène un groupe alkyle en $C_1$—$C_4$, un groupe alcényle en $C_4$ ou l'un d'entre eux est l'hydrogène tandis que l'autre est un groupe propen-2-yl ou encore $R_4$ et $R_5$ pris ensemble avec l'atome d'azote auquel ils sont liés forment un noyau cyclique à 5 ou 6 chaînons pouvant comporter un second atome d'azote, et

— X désigne l'anion d'un acide ou (I) est sous forme de bétaïne.

2. Dérivés selon la revendication 1, caractérisés en ce que $R_4$ et $R_5$ pris ensemble avec l'atome d'azote auquel ils sont liés forment l'un des cycles ci-après:

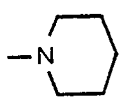 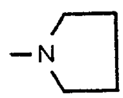 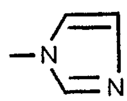 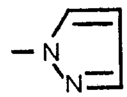

3. Dérivés selon l'une des revendications 1 ou 2, caractérisés en ce que les produits sont sous forme d'isomère syn.

4. Dérivés selon la revendication 3, caractérisé en que $R_1$ est —$C(CH_3)_2COOH$; $R_2 = R_3 = R_4 = R_5 = CH_3$; $X = CF_3COO$—, ledit composé étant le trifluoracétate de l'acide (amino-2 thiazolyl-4)-2 (carboxy-2 propyl-2 oxyimino)-2 acétamido)-7 (N,N,N',N'-tétraméthyl uronium thiomethyl)-3 cephéme-3 carboxyl-4 S-oxyde-1;

5. Dérivé selon la revendication 3, caractérisé en ce que $R_1$ est —$C(CH_3)_2COOH$; $R_2 = R_3 = CH_3$,

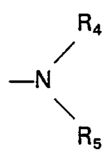 est le groupe de formule 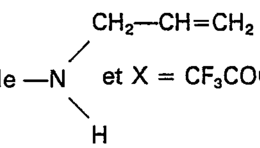 et $X = CF_3COO$—.

6. Dérivé selon la revendication 3, caractérisé en ce que $R_1$ est —$C(CH_3)_2COOH$; $R_2 = R_3 = CH_3$,

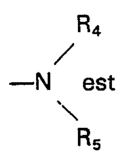 est le groupe de formule 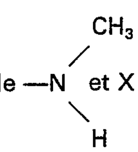 et $X = CF_3COO$—.

7. Dérivé selon la revendication 3, caractérisé en ce que $R_1$ est —$C(CH_3)_2COOH$; $R_2 = R_3 = CH_3$,

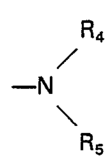

est le groupe —$NHC_2H_5$ et $X = CF_3COO$—.

8. Procédé de préparation des composés de formule (I) caractérisé en ce qu'on effectue la réaction de l'amino-7 bromométhyl-3 céphème-3 carboxylate de tertiobutyle-4 S-oxyde avec un acide de

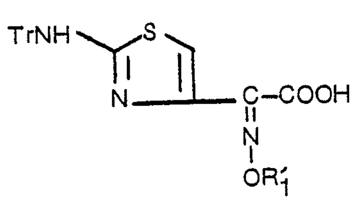

dans lequel Tr est un groupe, par exemple trityle, protecteur du groupe amino, $R'_1$ étant un ester labile du radical $R_1$ et dont la fonction acide à été activée par exemple par transformation en anhydride,

ladite réaction étant effectuée dans un solvant convenable tel que le diméthylformamide,
puis on élimine les groupes protecteurs présents sur la molécule obtenue et on fait réagir le produit obtenu avec une thiourée de formule

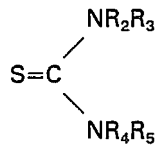
1

9. Médicaments, caractérisés en ce qu'ils contiennent au moins un dérivé des céphalosporines de formule (I).

10. Médicaments selon la revendication 9, utiles comme antibiotiques en médecine humaine et vétérinaire.

**Revendications pour l'état contractant: AT**

1. Procédé pour l'obtention de dérivés des céphalosporines de formule:

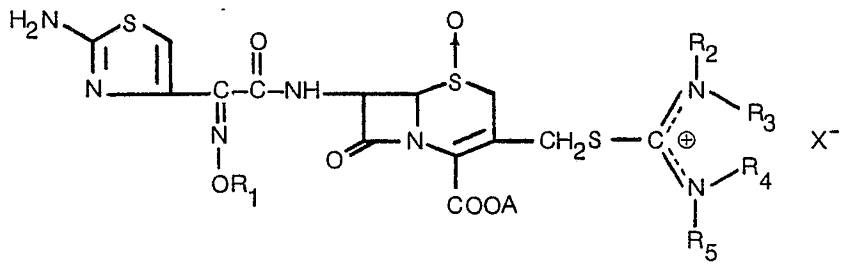
(I)

sous forme d'isomère syn, d'isomère anti ou d'un mélange de ces isomères, dans laquelle le groupe acide-COOA est sous forme acide ou sous forme de sel ou sous forme d'ester facilement hydrolysable,

— $R_1$ représente un groupe 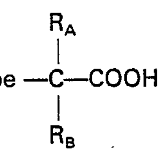

dans lequel $R_A$ et $R_B$ désignent chacun indépendamment l'hydrogène ou un groupe alkyle en $C_1$—$C_4$ de préférence un groupe méthyle, ou $R_A$ et $R_B$ pris ensemble avec l'atome de carbone, auquel ils sont liés, forment un groupe cyclobutyle;

— $R_2$ et $R_3$ désignent chacun indépendamment l'hydrogène, un groupe alkyle en $C_1$—$C_4$ ou un groupe alcényle en $C_4$;

— $R_4$ et $R_5$ désignent chacun indépendamment l'hydrogène, un groupe alkyle en $C_1$—$C_4$, un groupe alcényle en $C_4$ ou l'un d'entre eux est l'hydrogène tandis que l'autre est un groupe propen-2-yl ou encore $R_4$ et $R_5$ pris ensemble avec l'atome d'azote auquel ils sont liés, forment un noyau cyclique à 5 ou 6 chaînons pouvant comporter un second atome d'azote, et

— X désigne l'anion d'un acide ou (I) est sous forme de bétaïne.

caractérisé en ce qu'on effectue la réaction de l'amino-7 bromométhyl-3 céphème-3 carboxylate de tertiobutyle-4 S-oxyde avec un acide de formule:

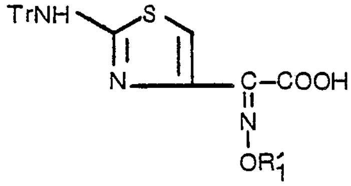

dans lequel Tr est un groupe, par exemple trityle, protecteur du groupe amino, $R'_1$ étant un ester labile du radical $R_1$ et dont la fonction acide à été activée par exemple par transformation en anhydride,

ladite réaction étant effectuée dans un solvant convenable tel que le diméthylformamide,

puis on élimine les groupes protecteurs présents sur la molécule obtenue et on fait réagir le produit obtenu avec une thiourée de formule

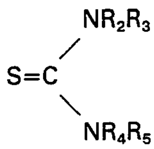 1

2. Procédé selon la revendication 1, caractérisé en ce que l'on fait réagir un composé de formule III tel que défini dans la revendication 1, avec une thiourée de formule (1), dans laquelle $R_2$ et $R_3$ sont tels que définis dans la revendication 1 et $R_4$ et $R_5$ pris ensemble avec l'atome d'azote auquel ils sont liés forment l'un des cycles ci-après:

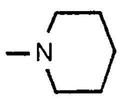 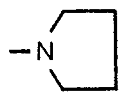 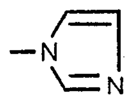 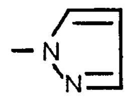

3. Procédé selon la revendication 1, caractérisé en ce que l'on utilise un composé de formule III sous forme d'isomère syn dans laquelle $R'_1$ est un ester labile du radical $R_1$ = —$C(CH_3)_2$ COOH et une thiourée de formule 1 dans laquelle $R_2 = R_3 = R_4 = R_5 = CH_3$ et que l'on obtient le composé de formule 1 correspondant sous forme de trifluoroacétate.

4. Procédé selon la revendication 1, caractérisé en ce que l'on utilise un composé de formule III sous forme d'isomère syn dans laquelle $R'_1$ est un ester labile du radical $R_1$ = —$C(CH_3)_2$ COOH et une thiourée de formule I dans laquelle $R_2 = R_3 = CH_3$ et

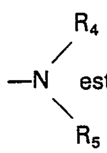 est l'un des groupes ci-après: 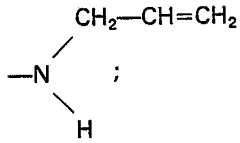 ; 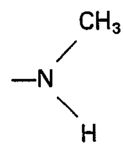 ou 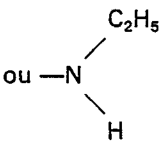

et que l'on obtient le composé de formule (I) correspondant sous forme de trifluoroacétate.

5. Utilisation des dérivés de céphalosporines obtenus par le procédé selon l'une quelconque des revendications 1 à 4 pour la préparation de médicaments.

6. Utilisation selon la revendication 5, pour la préparation d'antibiotiques, utilisables en médecine humaine ou vétérinaire.

**Patentansprüche für die Vertragsstaaten: BE CH DE FR GB IT LI LU NL SE:**

1. Cephalosporinderivate der Formel

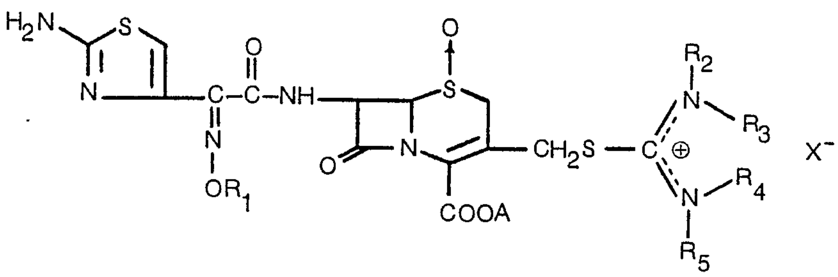 (I)

in syn-Isomerenform, anti-Isomerenform oder in Form einer Mischung dieser Isomere, worin die Gruppe Säure-COOA in Säureform oder in Salzform oder in Form eines leicht hydrolysierbaren Esters vorliegt,

— $R_1$ eine Gruppe 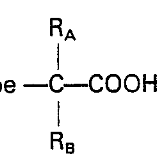 darstellt,

worin $R_A$ und $R_B$ jeweils unabhängig für Wasserstoff oder eine $C_1$—$C_4$-Alkylgruppe, vorzugsweise eine Methylgruppe, stehen, oder $R_A$ und $R_B$, zusammen mit dem Kohlenstoffatom, an das sie gebunden sind, eine Cyclobutylgruppe bilden;

— $R_2$ und $R_3$ jeweils unabhängig Wasserstoff, eine $C_1$—$C_4$-Alkylgruppe oder eine $C_4$-Alkenylgruppe bezeichnen;

— $R_4$ und $R_5$ jeweils unabhängig Wasserstoff, eine $C_1$—$C_4$-Alkylgruppe, eine $C_4$-Alkenylgruppe bezeichnen, oder eines davon Wasserstoff ist, während das andere für eine Propen-2-yl-gruppe steht, oder aber $R_4$ und $R_5$, zusammen mit dem Stickstoffatom, an das sie gebunden sind, einen zyklischen Kern mit 5 oder 6 Gliedern bilden, der ein zweites Stickstoffatom tragen kann, und

— X das Anion einer Säure darstellt oder (I) in Betainform vorliegt.

2. Derivate nach Anspruch 1, dadurch gekennzeichnet, daß $R_4$ und $R_5$, zusammen mit dem Stickstoffatom, an das sie gebunden sind, einen der nachstehenden Zyklen bilden:

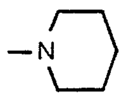 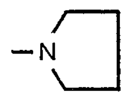 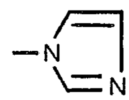 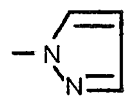

3. Derivate nach einem der Ansprüche 1 oder 2, dadurch gekennzeichnet, daß die Produkte in syn-Isomerenform vorliegen.

4. Derivate nach Anspruch 3, dadurch gekennzeichnet, daß $R_1$ für —$C(CH_3)_2COOH$ steht; $R_2 = R_3 = R_4 = R_5 = CH_3$; $X = CF_3COO^-$, welche Verbindung das Trifluoracetat der Säure, 2-(2-Amino-thiazol-4-yl)-7-(2-(2-carboxy-2-propyl-oxyimino)-acetamido)-3-(N,N,N',N'-tetramethyl-uronium-thiomethyl)-3-cephem-4-carboxyl-1-S-oxid, ist.

5. Derivat nach Anspruch 3, dadurch gekennzeichnet, daß $R_1$ für —$C(CH_3)_2COOH$ steht; $R_2 = R_3 = CH_3$,

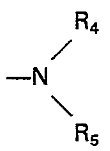 die Gruppe der Formel 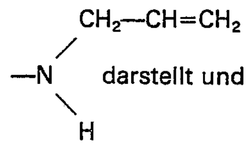 darstellt und $X = CF_3COO^-$ ist.

6. Derivat nach Anspruch 3, dadurch gekennzeichnet, daß $R_1$ für —$C(CH_3)_2COOH$ steht; $R_2 = R_3 = CH_3$,

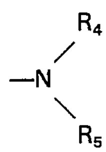 die Gruppe der Formel 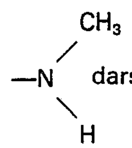 darstellt und $X = CF_3COO^-$.

7. Derivat nach Anspruch 3, dadurch gekennzeichnet, daß $R_1$ für —$C(CH_3)_2COOH$ steht; $R_2 = R_3 = CH_3$,

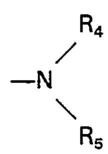

die Gruppe —$NHC_2H_5$ darstellt und $X = CF_3COO^-$.

8. Verfahren zur Herstellung der Verbindung der Formel (I), dadurch gekennzeichnet, daß man die Umsetzung von 7-Amino-3-brommethyl-3-cephem-4-tert. butylcarboxylat-S-oxid mit einer Säure der Formel

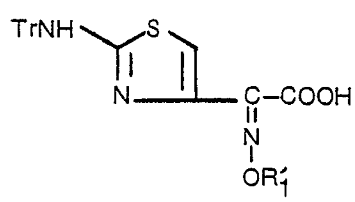

(III)

durchführt, worin Tr eine Schutzgruppe, beispielsweise Trityl, für die Aminogruppe ist, wobei $R'_1$ ein labiler Ester des Restes $R_1$ ist und dessen Säurefunktion beispielsweise durch Überführen in das Anhydrid aktiviert worden ist, welche Umsetzung in einem geeigneten Lösungsmittel, wie Dimethylformamid durchgeführt wird, man danach die am erhaltenen Molekül vorhandenen Schutzgruppen entfernt, und man das so erhaltene Produkt mit einem Thioharnstoff der Formel

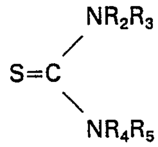 1

reagieren läßt.

9. Medikamente, dadurch gekennzeichnet, daß sie mindestens ein Cephalosporinderuvat der Formel (I) enthalten.

10. Medikament nach Anspruch 9, geeignet als Antibiotikum in der Human- und Veterinärmedizin.

**Patentansprüche für den Vertragsstaat: AT**

1. Verfahren zur Herstellung von Cephalosporinderivaten der Formel

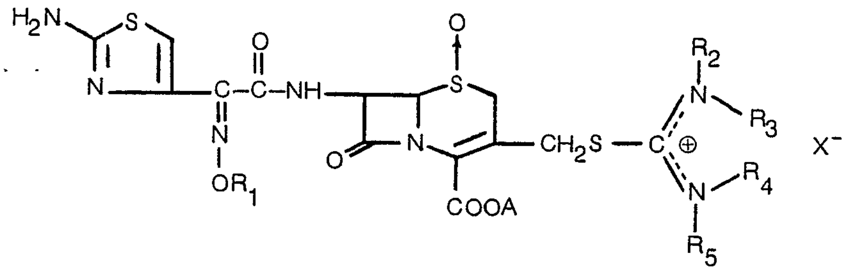 (I)

in syn-Isomerenform, anti-Isomerenform oder in Form einer Mischung dieser Isomere, worin die Gruppe Säure-COOA in Säureform oder in Salzform oder in Form eines leicht hydrolysierbaren Esters vorliegt,

— $R_1$ eine Gruppe 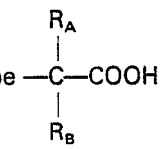 darstellt,

worin $R_A$ und $R_B$ jeweils unabhängig für Wasserstoff oder eine $C_1$—$C_4$-Alkylgruppe, vorzugsweise eine Methylgruppe, stehen, oder $R_A$ und $R_B$, zusammen mit dem Kohlenstoffatom, an das sie gebunden sind, eine Cyclobutylgruppe bilden;

— $R_2$ und $R_3$ jeweils unabhängig Wasserstoff, eine $C_1$—$C_4$-Alkylgruppe oder eine $C_4$-Alkenylgruppe bezeichnen;

— $R_4$ und $R_5$ jeweils unabhängig Wasserstoff, eine $C_1$—$C_4$-Alkylgruppe, eine $C_4$-Alkenylgruppe bezeichnen, oder eines davon Wasserstoff ist, während das andere für eine Propen-2-yl-gruppe steht, oder aber $R_4$ und $R_5$, zusammen mit dem Stickstoffatom, an das sie gebunden sind, einen zyklischen Kern mit 5 oder 6 Gliedern bilden, der ein zweites Stickstoffatom tragen kann, und

— X das Anion einer Säure darstellt oder (I) in Betainform vorliegt.

dadurch gekennzeichnet, daß man die Umsetzung von 7-Amino-3-brommethyl-3-cephem-4-tert. butylcarboxylat-S-oxid mit einer Säure der Formel

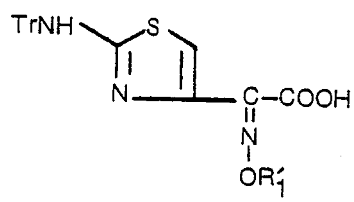

durchführt, worin Tr eine Schutzgruppe, beispielsweise Trityl, für die Aminogruppe ist, wobei $R'_1$ ein labiler Ester des Restes $R_1$ ist und dessen Säurefunktion beispielsweise durch Überführen in das Anhydrid aktiviert worden ist, welche Umsetzung in einem geeigneten Lösungsmittel, wie Dimethylformamid durchgeführt wird, man danach die am erhaltenen Molekül vorhandenen Schutzgruppen entfernt, und man das so erhaltene Produkt mit einem Thioharnstoff der Formel

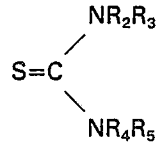 1

reagieren läßt.

2. Verfahren nach Anspruch 1, dadurch gekennzeichnet, daß man eine Verbindung der Formel III, wie in Anspruch 1 definiert, mit einem Thioharnstoff der Formel (1), worin $R_2$ und $R_3$ wie in Anspruch 1 definiert sind und $R_4$ und $R_5$, zusammen mit dem Stickstoffatom, an das sie gebunden sind, einen der nachstehenden Zyklen bilden:

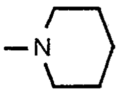 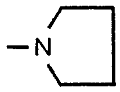 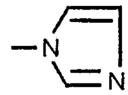 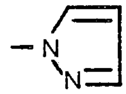

reagieren läßt.

3. Verfahren nach Anspruch 1, dadurch gekennzeichnet, daß man eine Verbindung der Formel III in syn-Isomerenform, worin $R'_1$ ein labiler Ester des Restes $R_1 = —C(CH_3)_2COOH$ ist, und einen Thioharnstoff der Formel 1, worin $R_2 = R_3 = R_4 = R_5 = CH_3$, verwendet, und daß man die entsprechende Verbindung der Formel 1 in Trifluoracetatform erhält.

4. Verfahren nach Anspruch 1, dadurch gekennzeichnet, daß man eine Verbindung der Formel III in syn-Isomerenform, worin $R'_1$ ein labiler Ester des Restes $R_1 = —C(CH_3)_2COOH$ ist, und einen Thioharnstoff der Formel I, worin $R_2 = R_3 = CH_3$ und

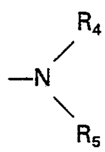

eine der nachstehenden Gruppen ist:

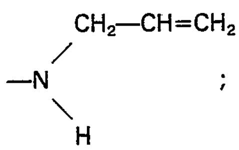 ; 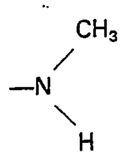 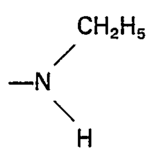

verwendet, und daß man die entsprechende Verbindung der Formel (I) in Trifluoracetatform erhält.

5. Verwendung der mit dem Verfahren nach einem der Ansprüche 1 bis 4 erhaltenen Cephalosporinderivate zur Herstellung von Medikamenten.

6. Verwendung nach Anspruch 5 zur Herstellung von Antibiotika, die in der Human- und Veterinärmedizin einsetzbar sind.

**Claims for the Contracting States: BE CH DE FR GB IT LI LU NL SE**

1. Cephalosporin derivatives of the formula:

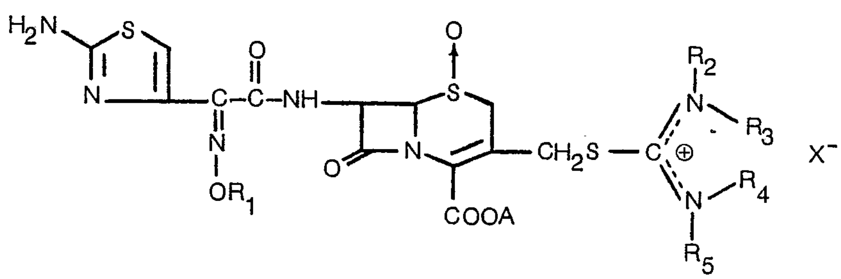 (I)

in the form of syn or anti isomers or a mixture of these isomers, and in which the acidic group —COOA is in acid form or in the form of salt or in the form of easily hydrolysable ester,

— $R_1$ represents a group: 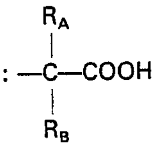

in which $R_A$ and $R_B$ each independently designates hydrogen or a $C_1$—$C_4$- alkyl group, preferably a methyl group, or $R_A$ and $R_B$, taken together with the carbon atom to which they are bonded, form a cyclobutyl group;

— $R_2$ and $R_3$ each independently designates hydrogen, a $C_1$—$C_4$- alkyl group or a $C_4$ alkenyl group;

— $R_4$ and $R_5$ each independently designates hydrogen, a $C_1$—$C_4$- alkyl group, a $C_4$ alkenyl group or one among them is hydrogen whereas the other is a 2-propen-yl group or still $R_4$ and $R_5$ taken together with the nitrogen atom to which they are bonded form a cyclic ring with 5 or 6 chains adapted to comprise a second nitrogen atom, and

— X designates an acid anion or (I) is in the form of betaine.

2. Derivatives according to claim 1, characterized in that $R_4$ and $R_5$ taken together with the atom of nitrogen to which they are bonded form one of the following cycles:

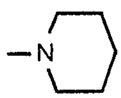 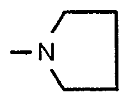 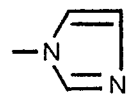 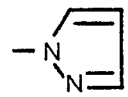

3. Derivatives according to any one of claims 1 or 2, characterized in that the products are in the form of syn isomer.

4. Derivative according to claim 3, characterized in that $R_1$ is —$C(CH_3)_2COOH$; $R_2 = R_3 = R_4 = R_5 = CH_3$; and X = $CF_3COO$—, said compound being the trifluoroacetate of 7-[2-(2-aminothiazol-4-yl)-2-(2-carboxy-2-propyloxyimino) acetamido]-3-[N,N,N',N'-tetramethyluroniumthiomethyl]-3-cephem-4-carboxylic acid.

5. Derivative according to claim 3, characterized in that $R_1$ is —$C(CH_3)_2COOH$; $R_2 = R_3 = CH_3$,

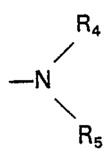 is the group of formula 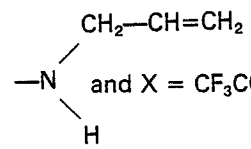 and X = $CF_3COO^-$.

6. Derivative according to claim 3, characterized in that $R_1$ is —$C(CH_3)_2COOH$; $R_2 = R_3 = CH_3$,

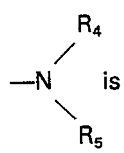 is the group of formula 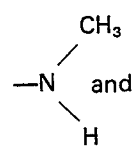 and X = $CF_3COO$—.

7. Derivative according to claim 3, characterized in that $R_1$ is —$C(CH_3)_2COOH$; $R_2 = R_3 = CH_3$;

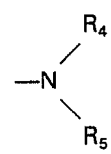

is the group of the formula —NH $C_2H_5$ and X = $CF_3COO$—.

8. Process for preparing compounds of formula (I), characterized in that it comprises the steps of reacting 7-amino 3-bromomethyl-3-cephem 4-tertiobutyl carboxylate S-oxide with an acid of formula:

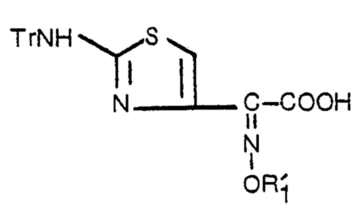

(III)

in which Tr is a protector group, for example a trityl group, of the amino group, $R'_1$ being a labile ester of the $R_1$ radical and of which the acid function has been activated, for example by conversion into anhydride,

said reaction being carried out in a suitable solvent such as dimethylformamide, then of removing the protector groups present on the resulting molecule, and the product obtained is caused to react with a thiourea of formula

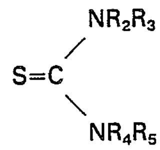

1

9. Drugs, characterized in that they contain at least one derivative of the cephalosporin of formula (I).

10. Drugs according to claim 9, useful as antibiotics in human or veterinary medicine.

**Claims for the Contracting State: AT**

1. Process for preparing the cephalosporin derivatives of the formula:

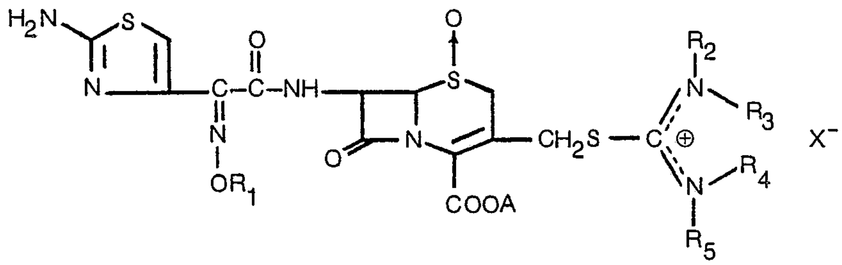

(I)

in the form of syn or anti isomers or a mixture of these isomers, and in which the acidic group —COOA is in acid form or in the form of salt or in the form of easily hydrolysable ester,

— $R_1$ represents a group: 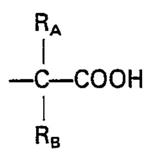

in which $R_A$ and $R_B$ each independently designates hydrogen or a $C_1$—$C_4$- alkyl group, preferably a methyl group, or $R_A$ and $R_B$, taken together with the carbon atom to which they are bonded, form a cyclobutyl group;

— $R_2$ and $R_3$ each independently designates hydrogen, a $C_1$—$C_4$- alkyl group or a $C_4$ alkenyl group;

— $R_4$ and $R_5$ each independently designates hydrogen, a $C_1$—$C_4$- alkyl group, a $C_4$ alkenyl group or one among them is hydrogen whereas the other is a 2-propen-yl group or still $R_4$ and $R_5$ taken together with the nitrogen atom to which they are bonded form a cyclic ring with 5 or 6 chains adapted to comprise a second nitrogen atom, and

— X designates an acid anion or (I) is in the form of betaine, characterized in that it comprises carrying out the reaction of 7-amino 3-bromomethyl-3-cephem-4-tertio-butylcarboxylate 2-oxide with an acid of formula:

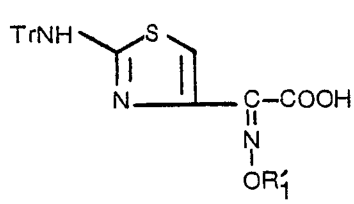

(III)

in which Tr is a group, for example a trityl group, protector of the amino group, $R'_1$ being a labile ester of the $R_1$ radical and of which the acid function has been activated, for example by conversion into anhydride, said reaction being carried out in a suitable solvent such as dimethylformamide, then the protector groups present on the resulting molecule, are removed and the product obtained is caused to react with a thiourea of formula

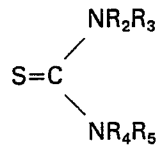 1

2. Process according to claim 1, characterized in that it comprises the step of reacting a compound of formula III such as defined in claim 1, with a thiourea of formula (1), in which $R_2$ and $R_3$ are such as defined in claim 1 and $R_3$ and $R_4$ taken together with the atom of nitrogen to which they are bonded form one of the following cycles:

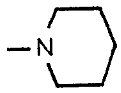 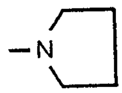 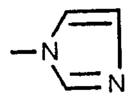 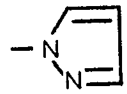

3. A process according to claim 1, characterized in that it comprises the step of using a compound of formula III in the form of syn isomer in which $R'_1$ is a labile ester of the radical $R_1 = —C(CH_3)_2\ COOH$ and a thiourea of formula (1) in which $R_2 = R_3 = R_4 = R_5 = CH_3$ and in that the corresponding compound of formula 1 in the form of trifluoroacetate is obtained.

4. Process according to claim 1, characterized in that it comprises the step of using a compound of formula III in the form of syn isomer in which $R'_1$ is a labile ester of the radical $R_1 = —C(CH_3)_2\ COOH$ and a thiourea of formula I in which $R_2 = R_3 = CH_3$ and

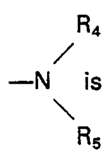 is one of the following groups: 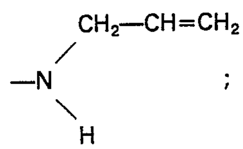 ; 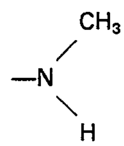 or 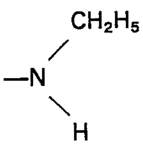

and in that the corresponding compound of formula (I) is obtained in the form of trifluoroacetate.

5. Use of cephalosporin derivatives obtained by the process according to any one of claims 1 to 4, for the preparation of drugs.

6. Use according to claim 5, for the preparation of antibiotics, usable in human and veterinary medicine.